(19) Europäisches Patentamt
European Patent Office
Office européen des brevets

(11) Veröffentlichungsnummer: **0 583 705 A1**

# (12) EUROPÄISCHE PATENTANMELDUNG

(21) Anmeldenummer: **93112633.8**

(22) Anmeldetag: **06.08.93**

(51) Int. Cl.[5]: **C07C 51/58**, C07C 17/12, C07C 63/70, C07C 25/13

(30) Priorität: **19.08.92 DE 4227371**

(43) Veröffentlichungstag der Anmeldung:
**23.02.94 Patentblatt 94/08**

(84) Benannte Vertragsstaaten:
**AT BE CH DE ES FR GB IT LI NL**

(71) Anmelder: **BAYER AG**

**D-51368 Leverkusen(DE)**

(72) Erfinder: **Beitzke, Bernhard, Dr.**
**Hasenfeld 38**
**D-51503 Rösrath-Forsbach(DE)**
Erfinder: **Diehl, Herbert, Dr.**
**Geibelstrasse 12**
**D-51373 Leverkusen(DE)**

## (54) Verfahren zur Herstellung von 2,3,4-trichlor-5-fluor-benzoyl-chlorid.

(57) Die Titelverbindung kann aus 2,4-Dichlor-5-fluor-benzotrichlorid hergestellt werden, indem man dieses zunächst in Gegenwart eines Friedel-Crafts-Katalysators chloriert, das entstandene Gemisch mit Wasser oder einer wasserabspaltenden Verbindung hydrolysiert und aus dem Hydrolysegemisch das 2,3,4-Trichlor-5-fluor-benzoylchlorid gewinnt.

EP 0 583 705 A1

Die vorliegende Erfindung betrifft ein Verfahren zur Herstellung der Titelverbindung durch Chlorierung von 2,4-Dichlor-5-fluor-benzotrichlorid in Gegenwart eines Katalysators, nachfolgende Hydrolyse und Gewinnung der Titelverbindung aus dem Hydrolysegemisch.

Die Titelverbindung ist ein Zwischenprodukt zur Synthese antibakteriell hochwirksamer Chinoloncarbonsäuren.

Aus DE-OS 36 31 906 ist es bekannt, die Titelverbindung dadurch herzustellen, daß man 2,4-Dichlor-5-fluorbenzoesäure in der 3-Position nitriert, zur Aminoverbindung hydriert, diese mit Kupferchlorid und Salzsäure diazotiert und die so erhaltene Trichlor-fluor-carbonsäure mit Thionylchlorid in das Säurechlorid überführt. Wegen seiner vielen Stufen ist dieser Weg sehr teuer.

Aus EP 431 373 ist ein weiterer Weg zur Herstellung der Titelverbindung bekannt, bei dem 1,2,3-Trichlor-4-fluor-benzol mit Tetrachlorkohlenstoff und Aluminiumchlorid umgesetzt und das Umsetzungsprodukt partiell zum Säurechlorid hydrolysiert wird Die Verwendung von Tetrachlorkohlenstoff ist jedoch aus arbeitshygienischen Gründen Einschränkungen unterworfen; ferner werden 200 Mol-% $AlCl_3$, bezogen auf die aromatische Ausgangsverbindung, eingesetzt. Die Ausbeute beträgt nur 61,8 % der theoretischen Ausbeute.

Weiterhin ist es aus DE-OS 39 12 263 bekannt, 2,4-Dichlor-5-fluor-benzoylchlorid mit Chlor zur Titelverbindung umzusetzen. Umsatz und Ausbeute sind bei diesem Verfahren jedoch nicht voll befriedigend; außerdem werden hohe Katalysatormengen zur Chlorierung benötigt. Die große Menge des nicht umgesetzten Ausgangsprodukts, die entstandenen Nebenprodukte und die hohe Katalysatormenge stören die Aufarbeitung.

Es bestand daher weiterhin die Aufgabe, einen Herstellungsweg zur Titelverbindung zu finden, der bezüglich der Ausbeute und der Wirtschaftlichkeit weiter verbessert war.

Es wurde nun gefunden, daß man das 2,4-Dichlor-5-fluor-benzotrichlorid mit guten Ausbeuten und Selektivitäten und sehr geringen Katalysatormengen chlorieren kann und das Reaktionsgemisch aus der Chlorierungsstufe ohne weitere Schritte hydrolysieren kann, wonach man ein Gemisch aus der Titelverbindung und mitentstandenem 2,4-Dichlor-5-fluor-benzoylchlorid erhält, das destillativ getrennt werden kann. Auch das mitentstandene 2,4-Dichlor-5-fluor-benzoylchlorid ist ein Zwischenprodukt für die Herstellung von Chinoloncarbonsäuren.

Es wurde ein Verfahren zur Herstellung von 2,3,4-Trichlor-5-fluor-benzoylchlorid gefunden, das dadurch gekennzeichnet ist, daß man 2,4-Dichlor-5-fluor-benzotrichlorid in Gegenwart eines Katalysators chloriert, das entstandene Gemisch mit Wasser oder wasserabspaltenden Substanzen hydrolysiert und aus dem Hydrolysegemisch das 2,3,4-Trichlor-5-fluorbenzoylchlorid gewinnt.

Die beiden Reaktionsstufen des erfindungsgemäßen Verfahrens lassen sich formelmäßig wie folgt darstellen, wobei in der zweiten Zeile berücksichtigt wurde, daß neben dem gewünschten Trichlor-Produkt auch noch Dichlor-Reaktionsprodukt enthalten ist, welches an der ersten Reaktionsstufe (Chlorierung) nicht teilgenommen hat:

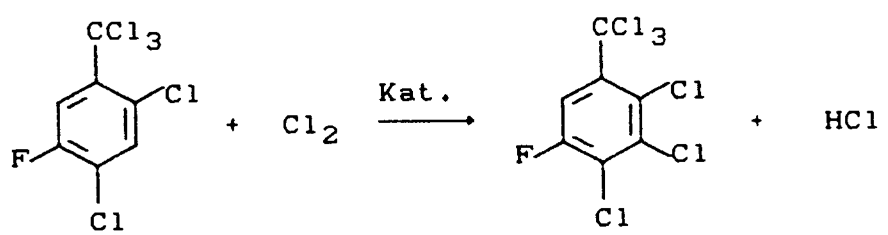


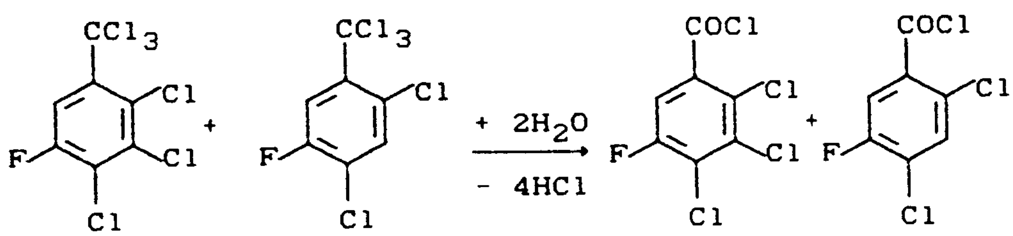


Das in der ersten Reaktionsstufe einzusetzende 2,4-Dichlor-5-fluor-benzotrichlorid kann beispielsweise gemäß DE-OS 31 42 856 durch Seitenkettenchlorierung aus 2,4-Dichlor-5-fluor-toluol hergestellt werden.

Als Chlorierungsmittel können erfindungsgemäß elementares Chlor oder chlorabgebende Substanzen, wie Sulfurylchlorid, bevorzugt elementares Chlor, eingesetzt werden.

Das erfindungsgemäße Verfahren wird in Gegenwart von Friedel-Crafts-Katalysatoren durchgeführt. Solche Katalysatoren sind beispielhaft Eisen(III)-chlorid, Titan(IV)-chlorid, Zinkchlorid, Galliumchlorid, Zinn-(IV)-chlorid, Aluminiumchlorid, Antimon(III)-chlorid, Antimon(V)-chlorid, Antimonylchlorid und Verbindungen, aus denen unter den erfindungsgemäßen Bedingungen solche Katalysatoren entstehen, beispielsweise metallisches Eisen, etwa in Form von Eisenspänen, Eisen(II)-chlorid und weitere dem Fachmann bekannte. In bevorzugter Weise wird Eisen(III)-chlorid oder metallisches Eisen eingesetzt. In weiterhin bevorzugter Weise wird Antimon(III)-chlorid oder eine Antimon-Verbindung, die unter den erfindungsgemäßen Bedingungen in Antimon(III)-chlorid umgewandelt wird, eingesetzt.

In einer bevorzugten Variante wird zu dem Friedel-Crafts-Katalysator ein Co-Katalysator eingesetzt, beispielsweise elementarer Schwefel, Schwefelchloride, wie $S_2Cl_2$ oder $SCl_2$, elementares Iod oder elementares Brom. Beispielsweise wird Eisen(III)-chlorid häufig gemeinsam mit elementarem Schwefel oder elementarem Iod als Chlorierungskatalysator eingesetzt. Weitere Co-Katalysatoren entstammen der Gruppe der höheren Alkohole, beispielsweise Lorol und der Gruppe der Glykolether. Es handelt sich um höhere Fettalkohole mit 10 bis 18 C-Atomen, z.B. n-Decanol, n-Hexadecanol u.a..

Das erfindungsgemäße Verfahren kann auch in einem unter den Reaktionsbedingungen inerten Lösungsmittel durchgeführt werden. Solche Lösungsmittel sind dem Fachmann bekannt; als besonders günstig haben sich Phosphoroxychlorid und Thionylchlorid, aber auch Chloraromaten, erwiesen. In bevorzugter Weise wird jedoch ohne Lösungsmittel gearbeitet.

Chlor (elementares oder aus einer Verbindung abgespaltenes) wird in einer Menge von 30 bis 300 Mol-%, bezogen auf den Einsatzstoff, bevorzugt von 50 bis 200 Mol-%, besonders bevorzugt von 70 bis 150 Mol-% eingesetzt. Die Katalysatormenge beträgt 0,05 bis 10 Mol-%, bezogen auf den Einsatzstoff, bevorzugt 1 bis 5 Mol-%, besonders bevorzugt 2 bis 4 Mol-%. Der Co-Katalysator wird in einer Menge von 10 bis 200 Mol-%, bezogen auf die Katalysatormenge, eingesetzt.

Die Temperatur für die erste Verfahrensstufe beträgt 20 bis 180 °C. Bei hohen Temperaturen kommt es verstärkt zu Nebenreaktionen; bei tiefen Temperaturen ist die Chloriergeschwindigkeit, zusätzlich in Abhängigkeit von der Menge des Katalysators, sehr gering. In bevorzugter Weise wird daher bei 70 bis 160 °C, in besonders bevorzugter Weise bei 90 bis 140 °C chloriert.

Die Chlorierung wird im allgemeinen bei Normaldruck durchgeführt. Es ist jedoch möglich, unter Druck, beispielsweise bei bis zu 5 bar, zu arbeiten und kontinuierlich oder absatzweise den entstandenen Chlorwasserstoff zu entspannen.

Die Reaktionszeit für die Chlorierung richtet sich nach der Art und Menge des Katalysators sowie nach der Temperatur. Vor allem ist die Reaktionszeit aber von der Ansatzgröße abhängig. Unter den bevorzugten Reaktionsbedingungen liegt sie für einen 0,5 bis 2 l-Ansatz typischerweise zwischen 6 und 15 Stunden.

Die erste Stufe des erfindungsgemäßen Verfahrens kann diskontinuierlich, beispielsweise in einem Rührkessel oder kontinuierlich, beispielsweise in einer Kesselkaskade oder einer Blasensäule, durchgeführt werden; bevorzugt wird diskontinuierlich gearbeitet. Hierzu werden Ausgangsstoff, Katalysator, gegebenenfalls Co-Katalysator und gegebenenfalls Lösungsmittel vorgelegt und auf die gewünschte Reaktionstemperatur aufgeheizt. Dann wird das Chlorierungsmittel eingeleitet, bevorzugt über ein Tauchrohr unter starkem Rühren. Der Reaktionsfortschritt wird durch Probennahme und gaschromatographische Analyse festgestellt. Die Geschwindigkeit der Chloreinleitung kann so gelenkt werden, daß praktisch kein Chlor mit dem Abgas durchschlägt.

In bevorzugter Weise wird die Chlorierungsreaktion vor dem vollständigen Umsatz abgebrochen, um die Bildung unerwünuchter Nebenprodukte zu unterdrücken. So wird im allgemeinen bis zu einem Umsatz von 20 bis 90 Mol-%, bevorzugt 30 bis 70 Mol-%, besonders bevorzugt 40 bis 60 Mol-% des Ausgangsproduktes chloriert.

In einer weiteren vorteilhaften Variante wird die Chlorierungsreaktion in Gegenwart einer Menge von 0,01 bis 10 Gew.-% an $CaCl_2$, bezogen auf den Ausgangsstoff, durchgeführt. Hierbei wird eine Erhöhung der Ausbeute festgestellt, die offenbar auf die Unterdrückung nicht identifizierter Nebenprodukte zurückzuführen ist.

Wird die Isolierung des 2,3,4-Trichlor-5-fluor-benzotrichlorids in reiner Form gewünscht, so wird nach Ende der Chlorierung fraktioniert destilliert, wobei man das nicht umgesetzte 2,4-Dichlor-5-fluorbenzotrichlorid als Vorlauf abtrennt.

In der zweiten Stufe wird das Zwischenprodukt 2,3,4-Trichlor-5-fluor-benzotrichlorid, nach destillativer Abtrennung oder bevorzugt ohne eine solche Abtrennung im Reaktionsgemisch der ersten Stufe des erfindungsgemäßen Verfahrens, durch Zusatz von Wasser oder wasserabspaltenden Mitteln, wie Ameisensäure, hydrolysiert. Das zur Hydrolyse einzusetzende Wasser kann auch das Verdünnungswasser eines

anderen Stoffes sein, beispielsweise in Form von nicht konzentrierter Schwefelsäure. Wasser oder eine wasserabgebende Substanz wird im wesentlichen in äquimolarer Menge, bezogen auf den Ausgangsstoff, eingesetzt, beispielsweise in einer Menge von 0,8 bis 1,2 Mol, bevorzugt 0,9 bis 1,1 Mol, besonders bevorzugt 0,95 bis 1,05 Mol $H_2O$ pro Mol Ausgangsstoff.

Die Hydrolyse wird in Gegenwart oder bei Abwesenheit eines gegen die Hydrolyse inerten Lösungsmittels durchgeführt. Als geeignete Lösungsmittel haben sich beispielsweise Chlorbenzol oder eines der isomeren Dichlorbenzole erwiesen. In bevorzugter Weise wird bei Abwesenheit eines Lösungsmittels gearbeitet.

Die Hydrolyse kann rein thermisch oder in Gegenwart eines Katalysators durchgeführt werden. Solche Katalysatoren gehören der Gruppe der Lewis- und Brönsted-Säuren an. Beispiele hierfür sind: $FeCl_3$, $ZnCl_2$, $ZrOCl_2$, $H_2SO_4$, $SbCl_3$, $AlCl_3$ und andere. Die Hydrolyse von Benzotrichloriden zu Benzoylchloriden ist im Prinzip bekannt (Ullmanns Encyclopädie der technischen Chemie, 4. Auflage, Band 8 (1974), S. 373). Der Zusatz eines der genannten Katalysatoren bezieht sich vor allem auf die Hydrolyse eines zwischenisolierten 2,3,4-Trichlor-5-fluor-benzotrichlorides. Für den bevorzugten Fall, daß das Reaktionsgemisch aus der Chlorierungsstufe ohne Isolierung des Zwischenproduktes der Hydrolyse unterworfen wird, ist es ausreichend, die Wirksamkeit des Chlorierungskatalysators, der ebenfalls eine Lewis-Säure darstellt, auch für die Hydrolysestufe zu benutzen. Für den Fall, daß ein zwischenisoliertes Material hydrolysiert wird, gelten für die Mengen des zuzusetzenden Katalysators die oben gemachten Angaben auch für die Hydrolysestufe.

Die Hydrolyse wird bei einer Temperatur von 20 bis 200 °C, bevorzugt 80 bis 160 °C durchgeführt.

Das isolierte Zwischenprodukt oder das gesamte Reaktionsgemisch der ersten Stufe wird hierzu vorgelegt, auf die Reaktionstemperatur gebracht, woraufhin die genannte Wassermenge eindosiert wird. Die Dosiergeschwindigkeit des Wassers oder der wasserabspaltenden Substanz richtet sich nach der Temperatur, der Wärmeabfuhr (exotherme Reaktion) und der Gasabfuhr. Auch in der zweiten Stufe wird der Reaktionsfortschritt durch gaschromatographische Analyse von entnommenen Proben verfolgt. Die Aufarbeitung nach der zweiten Reaktionsstufe erfolgt durch fraktionierte Destillation.

In der bevorzugten Reaktionsvariante wird unter Ausnutzung des in bevorzugter Weise nicht vollständigen Umsatzes in der Chlorierungsstufe über beide Reaktionsstufen ein Gemisch aus 2,3,4-Trichlor-5-fluor-benzoylchlorid und 2,4-Dichlor-5-fluor-benzoylchlorid hergestellt. Bei dieser Variante wird also das infolge unvollständigen Umsatzes unverändert gebliebene Ausgangsmaterial 2,4-Dichlor-5- fluor-benzotrichlorid in der Hydrolysestufe miterfaßt, so daß die beiden genannten Benzoylchloride gleichzeitig hergestellt werden. Deren Trennung erfolgt durch fraktionierte Destillation. Auch dieses mitentstandene 2,4-Dichlor-5-fluor-benzoylchlorid ist ein Zwischenprodukt für die Herstellung von Chinoloncarbosäuren.

<u>Beispiel 1</u>

In einem 2 l-Kolben mit Rührer, Kondensator mit Gasabgang und Gaseinleitungsrohr für Chlorgas wurden vorgelegt:
2600 g 2,4-Dichlor-5-fluor-benzotrichlorid, 99 %,
1,5 g Eisen(III)chlorid und
0,5 g Schwefel.

Das Reaktionsgemisch wurde auf 130 °C erwärmt, wonach mit dem Einleiten von Chlor begonnen wurde. Es wurden in 9 Stunden insgesamt 600 g $Cl_2$ eingeleitet. Danach wurde eine Probe gezogen. Das Gaschromatogramm zeigte an: 44,6 % 2,4-Dichlor-5-fluor-benzotrichlorid und 46,0 % 2,3,4-Trichlor-5-fluor-benzotrichlorid, Rest: Nebenprodukte.

Nun wurde bei 140 °C in ca. 8 Stunden eine Menge von 150 g Wasser unter die Oberfläche dosiert.

Danach wurde der Ansatz über eine Kolonne fraktioniert destilliert. Es wurden erhalten;
880 g 2,4-Dichlor-5-fluor-benzoylchlorid (42,5 % der theoretischen Ausbeute),
980 g 2,3,4-Trichlor-5-fluor-benzoylchlorid (41,0 %), Siedepunkt 121 °C bei 12 mbar,
106 g 2,3,4,6-Tetrachlor-5-fluor-benzoylchlorid (3,9 %),
120 g 2,3,4-Trichlor-5-fluorbenzotrichlorid (4,2 % d.Th.) und
85 g Destillationsrückstand (Siedebeginn über 220 °C bei 12 mbar).

<u>Beispiel 2</u>

In einem 1 l-Kolben mit Rührer, Kondensator mit Gasabgang und Gaseinleitrohr wurden vorgelegt:
858 g 2,4-Dichlor-5-fluor-benzotrichlorid und
0,5 g Eisen(III)chlorid.

Das Reaktionsgemisch wurde auf 140 °C geheizt. Zwischen 140 °C und 180 °C wurden in 9 Stunden insgesamt 302 g Chlor eingeleitet. Danach wurde über eine Kolonne fraktioniert destilliert. Es wurden erhalten:
70,6 g Pentachlorfluorbenzol (8,8 % der theoretischen Ausbeute)
80,6 g 2,4-Dichlor-5-fluor-benzotrichlorid (9,5 %),
Siedepunkt 132-134 °C/8 mbar,
514 g 2,3,4-Trichlor-5-fluorbenzotrichlorid (54,1 %),
Siedepunkt 155 °C/8 mbar und
51,5 g 2,3,4,6-Tetrachlor-5-fluorbenzotrichlorid (4,9 %).

Beispiele 3 bis 9

In einem 1 l-Kolben mit Rührer, Kondensator mit Gasabgang sowie mit Gaseinleitrohr wurden
858 g 2,4-Dichlor-5-fluor-benzotrichlorid (98,8 %ig)
vorgelegt. Es wurde in Gegenwart verschiedener Katalysatoren chloriert (siehe Tabelle). Nach verschiedenen Chlormengen wurden Proben gezogen und gaschromatographisch untersucht. Die Ergebnisse sind in der Tabelle wiedergegeben.

<u>Tabelle</u> (Beispiele 3 bis 8): Chlorierung von 2,4-Dichlor-5-fluor-benzotrichlorid mit verschiedenen Katalysatoren

| Beispiel | Katalysator I | Katalysator II | Zeit h | Temp. °C | Chlor g | Chlor Mol | a Mol: 268,3 % | b Mol: 282,3 % | c Mol: 316,8 % | d Mol: 333,2 % | e Mol: 351,2 % |
|---|---|---|---|---|---|---|---|---|---|---|---|
| 3 | (Mol :162)<br>Fe(III)Cl3<br>0,5 g<br>0,1 Mol% | - | 9 | 140 - 180 | 127<br>213<br>302 | 1,79<br>3,00<br>4,25 | -<br>4,24<br>10,1 | 55,9<br>30,3<br>0,21 | 38,5<br>57,7<br>63,1 | 0,39<br>0,36<br>0,34 | 1,29<br>3,12<br>5,18 |
| 4 | (Mol :162)<br>Fe(III)Cl3<br>0,5 g<br>0,1 Mol% | (Mol :32)<br>Schwefel<br>0,17 g<br>0,02 % | 9 | 140 - 160 | 170<br>213<br>240 | 2,39<br>3,00<br>3,38 | 1,17<br>2,15<br>2,99 | 43,6<br>31,5<br>24,9 | 47,1<br>54,6<br>57,4 | 0,78<br>1,05<br>1,27 | 2,22<br>3,61<br>4,78 |
| 5 | (Mol :162)<br>Fe(III)Cl3<br>0,5 g<br>0,1 Mol% | (Mol :228)<br>Lorol,tchn<br>2,82 g<br>4mol/mol | 13 | 140 - 180 | 108<br>162<br>219<br>250 | 1,52<br>2,28<br>3,08<br>3,52 | 1,27<br>2,18<br>3,65<br>5,20 | 66,1<br>55,7<br>44,4<br>37,2 | 27,4<br>35,5<br>42,4<br>45,6 | 0,57<br>0,73<br>1,02<br>1,14 | -<br>-<br>0,45<br>0,38 |
| 6 | (Mol :162)<br>Fe(III)Cl3<br>0,5 g<br>0,1 Mol% | (Mol :254)<br>Jod<br>0,17 g<br>0,02 % | 7 | 140 - 180 | 146<br>217 | 2,06<br>3,06 | 1,43<br>3,92 | 49,4<br>30,3 | 42,2<br>54,0 | 0,70<br>1,10 | 1,38<br>2,35 |
| 7 | - | (Mol :228)<br>Sb(III)Cl3<br>2 x 0,68 g<br>0,2 Mol% | 17 | 140 - 160 | 4h /138<br>10h /184<br>15h /276<br>319 | 1,94<br>2,59<br>3,89<br>4,49 | 0,88<br>1,72<br>3,93<br>7,68 | 59,7<br>48,0<br>27,5<br>15,4 | 33,4<br>39,9<br>56,8<br>55,4 | 0,81<br>1,26<br>1,94<br>2,25 | 0,44<br>0,77<br>1,66<br>2,70 |
| 8 | (Mol :133)<br>Al(III)Cl 3<br>2 x 0,40 g<br>0,2 Mol% | - | 15 | 70 - 180 | 102<br>152 | 1,44<br>2,14 | 0,28<br>0,44 | 84,7<br>81,4 | 12,2<br>14,7 | 0,74<br>0,82 | -<br>- |

a) Pentachlorfluorbenzol

b) Edukt

c) Produkt = 2,3,4-Trichlor-5-fluor-benzotrichlorid

d) Tetrachlorbenzotrichlorid

e) 2,3,4,6-Tetrachlor-5-fluor-benzotrichlorid

## Patentansprüche

1. Verfahren zur Herstellung von 2,3,4-Trichlor-5-fluor-benzoylchlorid, dadurch gekennzeichnet, daß man 2,4-Dichlor-5-fluor-benzotrichlorid in Gegenwart eines Friedel-Crafts-Katalysators chloriert, das entstandene Zwischenprodukt durch Wasser oder wasserabspaltende Substanzen hydrolysiert und aus dem Hydrolysegemisch das 2,3,4-Trichlor-5-fluor-benzoylchlorid gewinnt.

2. Verfahren nach Anspruch 1, dadurch gekennzeichnet, daß als Katalysator für die Chlorierung Eisen oder Eisen(III)-chlorid verwendet wird.

3. Verfahren nach Anspruch 1, dadurch gekennzeichnet, daß als Katalysator für die Chlorierung Antimon-(III)-chlorid oder eine Antimonverbindung verwendet wird, die unter den Reaktionsbedingungen Antimon(III)-chlorid bildet.

4. Verfahren nach Anspruch 1, dadurch gekennzeichnet, daß als Co-Katalysatoren Schwefel, Schwefelchloride, Iod oder Brom verwendet werden.

5. Verfahren nach Anspruch 1, dadurch gekennzeichnet, daß die Chlorierung nur bis zu einem Teilumsatz von 20 bis 90 Mol-%, bevorzugt 30 bis 70 Mol-%, besonders bevorzugt 40 bis 60 Mol-% des Ausgangsstoffes geführt wird.

6. Verfahren nach Anspruch 1, dadurch gekennzeichnet, daß das Reaktionsgemisch der ersten Reaktionsstufe ohne Isolierung des Zwischenproduktes der Hydrolyse unterworfen wird.

7. Verfahren nach Anspruch 1, dadurch gekennzeichnet, daß bei der Chlorierung Calciumchlorid in einer Menge von 0,01 bis 10 Gew.-%, bezogen auf eingesetztes Dichlor-fluor-benzotrichlorid, zugesetzt wird.

8. Verfahren nach Anspruch 5, dadurch gekennzeichnet, daß neben 2,3,4-Trichlor-5-fluor-benzoylchlorid gleichzeitig 2,4-Dichlor-5-fluor-benzoylchlorid hergestellt wird, indem man das eingesetzte 2,4-Dichlor-5-fluor-benzotrichlorid nur bis zu einem Teilumsatz chloriert, das Reaktionsgemisch hydrolysiert und anschließend die Produkte durch Destillation trennt.

9. Verfahren nach Anspruch 1, dadurch gekennzeichnet, daß die Hydrolyse in Gegenwart eines Katalysators aus der Gruppe der Lewis- und Brönsted-Säuren durchgeführt wird.

10. Verfahren nach Anspruch 9, dadurch gekennzeichnet, daß man als Katalysator für die Hydrolyse den aus der Chlorierungsreaktion im Gemisch befindlichen benutzt.

Europäisches Patentamt

# EUROPÄISCHER RECHERCHENBERICHT

Nummer der Anmeldung

EP 93 11 2633

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | CHEMICAL ABSTRACTS, vol. 117, 1992, Columbus, Ohio, US; abstract no. 48085t, * Zusammenfassung * | 1,2,9 | C07C51/58 C07C17/12 C07C63/70 C07C25/13 |
| Y | * Zusammenfassung * & REGISTRY (Datenbank, STN) RN: 86522-8-5, 115549-05-8, 136364-62-0 & DATABASE WPI Week 9213, Derwent Publications Ltd., London, GB; AN 92-102797 * Zusammenfassung * & JP-A-04 049 264 (ASAHI GLASS KK) --- | 3,4,6,7, 9,10 | |
| Y | CHEMICAL ABSTRACTS, vol. 114, 1991, Columbus, Ohio, US; abstract no. 121705q, * Zusammenfassung * & BR-A-8 900 736 (DEFENSA-INDUSTRIA DE DEFENSIVOS AGRICOLAS SA) --- | 3,4 | |
| | | | RECHERCHIERTE SACHGEBIETE (Int. Cl.5 ) |
| Y | DE-A-2 644 641 (BAYER AG) * Ansprüche * --- | 4 | C07C |
| Y | EP-A-0 024 516 (BAYER AG) * Seite 4, Zeilen 7-24; Seite 8, Zeile 20 - Seite 9, Zeile 20 * --- | 6,9,10 | |
| Y | US-A-4 401 623 (T.J. GIACOBBE ET AL) * Ansprüche * ----- | 7 | |

Der vorliegende Recherchenbericht wurde für alle Patentansprüche erstellt

| Recherchenort | Abschlußdatum der Recherche | Prüfer |
|---|---|---|
| BERLIN | 29 NOVEMBER 1993 | VAN AMSTERDAM L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.......................................................
& : Mitglied der gleichen Patentfamilie, übereinstimmendes Dokument